# EUROPÄISCHE PATENTSCHRIFT

(11) **EP 1 904 122 B1**
(45) Veröffentlichungstag und Bekanntmachung des Hinweises auf die Patenterteilung: **08.02.2012**
(21) Anmeldenummer: 06742405.1
(22) Anmeldetag: 09.06.2006
(51) Int. Cl.: A61M 1/36, A61M 1/16

(54) **Dialysegerät und Verfahren zur Überprüfung einer Messeinheit**
Dialysis apparatus and method for checking a measuring unit
Appareil de dialyse et procédé de contrôle d'un moyen de mesure

(30) Priorität: 24.06.2005 DE 102005029709
(43) Veröffentlichungstag der Anmeldung: 02.04.2008
(73) Patentinhaber: Fresenius Medical Care Deutschland GmbH, 61352 Bad Homburg (DE)
(72) Erfinder: ZHANG, Wei, 97464 Niederwerm (DE); SCHULTE, Elke, 97424 Schweinfurt (DE)
(74) Vertreter: Vièl, Christof
(86) Internationale Anmeldenummer: PCT/DE2006/001000
(87) Internationale Veröffentlichungsnummer: WO 2006/136134

(56) Entgegenhaltungen:
- EP-A- 0 911 044
- EP-A- 1 273 315
- WO-A-02/35979
- US-A1- 2005 096 557

## Beschreibung

Die Erfindung betrifft ein Verfahren zur Überprüfung wenigstens einer Messgröße nach dem Oberbegriff des Patentanspruchs 1 sowie ein Dialysegerät nach dem Oberbegriff des Patentanspruchs 6.

Aus der EP 0 911 044 A1 (entspricht US-PS 6,736,789) ist es bekannt, den Blutdruck des Patienten aus einem Signal zu generieren, das der Pulswellenlaufzeit entspricht. Dazu wird mittels eines Elektrokardiogramms beginnend vom Zeitpunkt eines Herzschlags gemessen, wie lange es dauert, bis ein Druckpuls an einer vom Herzen entfernten Stelle am Patienten (beispielsweise an einem Finger) gemessen werden kann. Die Messung kann mittels eines Drucksensors erfolgen. Alternativ oder zusätzlich kann die Messung auch mit einem Photoplethysomographen erfolgen. Die Messsignale werden der Auswerteeinheit kabelgebunden zugeführt. Zwischen dem Blutdruck des Patienten und der Pulswellenlaufzeit besteht ein linearer Zusammenhang. Es ist weiterhin beschrieben, mittels einer herkömmlichen Manschettenmessung parallel zur Messung der Pulswellenlaufzeit unmittelbar den Blutdruck zu bestimmen. Aus zusammengehörenden Wertepaaren zweier Messungen lassen sich die Parameter der linearen Gleichung bestimmen. Nach der Bestimmung der Parameter kann aus der Messung der Pulswellenlaufzeit unmittelbar der Blutdruck errechnet werden.

Damit kann von dem Dialysegerät während der Dialysebehandlung selbsttätig der Blutdruck gemessen werden. Eine hypotone Episode während der Dialysebehandlung kann sich plötzlich schnell entwickeln und jederzeit vorkommen und bis zur Bewusstlosigkeit des Patienten bzw. zur Notwendigkeit des Abbruchs der Behandlung führen. Das Wohlbefinden und der Gesundheitszustand des Patienten werden dadurch hegativ beeinflusst.

Durch die Ultrafiltration während der Dialysebehandlung kommt es zu einer Abnahme des Plasmavolumens. Falls der Organismus es nicht schafft, das Plasmavolumen aus dem interstitiellen Raum aufzufüllen, sinkt der Füllungsdruck des Herzens und der Blutdruck fällt. Dieses Risiko ist um so gröBer, je höher die Filtrationsraten sind.

Um den Abfall des Blutdrucks frühzeitig zu erkennen und/oder zu vermeiden, sind verschiedene Wirkungsweisen von Dialysegeräten bekannt. Zum einen gibt es Monitore, die das relative Blutvolumen während der Dialysebehandlung messen und ggf. die Ultrafiltration regeln. Zum anderen gibt es Monitore, die Blutdruckänderungen eines Patienten durch die Erfassung der Pulswellenlaufzeit (PTT) kontinuierlich überwachen und ggf. die Ultrafiltration regeln. Die Zeit eines Druckpulses, die dieser zwischen zwei Punkten entlang eines Gefäßes im Patienten benötigt, ist eine Funktion des Blutdrucks. Diese Zeit lässt sich vergleichsweise einfach ermitteln. Als Startsignal kann ein EKG-Puls verwendet werden., während als Stopp-Signal ein optischen Pulsmessgerät an einer vom Herz entfernten Stelle verwendet werden kann. Diese Vorgehensweise ist beispielsweise in der bereits erwähnten EP 0 911 044 A1 (entspricht US-PS 6,736,789) beschrieben.

Weiterhin ist es bekannt, den Blutdruck mittels einer Manschettenmessung zu überwachen. Hierbei kann es für den Patienten als unangenehm empfunden werden, dass in regelmäßigen Abständen (z. B. alle 30 Minuten) die Manschette mit Druck beaufschlagt werden muss, um die Messung durchführen zu können. Ein weiterer und entscheidender Nachteil der Manschettenmessung liegt darin, das ein rasanter Blutdruckabfall wegen der Diskontinuität der Messung schwer zu erfassen ist.

Die PTT-Messeinheiten sind durchweg kabelgebunden mit dem Dialysegerät verbunden, mit dem der Patient behandelt werden soll. Eine solche Kabelverbindung beschränkt jedoch die Beweglichkeit des Patienten und kann bei den Bewegungen auch Signalverfälschungen hervorrufen.

Es ist auch bekannt, bei Messsonden die Kabelverbindung durch eine Funkverbindung zu ersetzen. Diese kann beispielsweise aufbauend auf dem Bluetooth-Standard basieren. Eine entsprechende Ausgestaltung ist beispielsweise aus der US 2005/0096557 A1 bekannt. Ausgehend von diesem Stand der Technik liegt der Erfindung das nachfolgende Problem zu Grunde.

Der vorliegenden Erfindung liegt das Problem zu Grunde, automatisch geräteseitig vom Dialysegerät aus die Zuordnung einer drahtlos Messdaten an das Dialysegerät übermittelnden Messeinheit zu dem Dialysegerät überprüfen zu können.

Insbesondere in ärztlichen Einrichtungen, in denen mehrere derartige Dialysegeräte gleichzeitig betrieben werden, erweist sich eine solche Überprüfung als vorteilhaft, um geräteseitig erkennen zu können, wenn die drahtlos Messdaten übermittelnde Messeinheit nicht ordnungsgemäß funktioniert oder eventuell falsch angeschlossen ist.

Diese Aufgabe wird nach der vorliegenden Erfindung durch ein Verfahren gemäß Anspruch 1 gelöst, wonach bei einem Dialysegerät mit einer dem Dialysegerät zugeordneten Messeinheit, bei der die Datenübertragung von der Messeinheit zu dem Dialysegerät drahtlos erfolgt, von der Messeinheit wenigstens eine Messgröße erfasst und an das Dialysegerät übertragen wird, die von einer weiteren, mit dem Dialysegerät fest verbundenen Messeinrichtung ebenfalls erfasst wird, wobei aus einem Vergleich der mit der Messeinheit sowie der Messeinrichtung erfassten wenigstens einen Messgröße auf die Zuordnung und/oder Funktion der Messeinheit geschlossen wird.

Vorteilhaft wird also die Messeinheit durch das Dialysegerät überwacht und kontrolliert, indem eine Messgröße der drahtlos an das Dialysegerät angekoppelten Messeinheit von dem Dialysegerät nochmals unabhängig erfasst wird. Durch einen Vergleich kann überprüft werden, ob die richtige Messeinheit angekoppelt ist und/oder ob eventuell eine Funktionsstörung vorliegt.

Die Messeinrichtung kann mit dem Dialysegerät fest verbunden sein, indem diese baulich in das Dialysegerät integriert ist oder auch, indem diese mittels eines Kabelverbindung an das Dialysegerät angeschlossen wird.

Bei der Ausgestaltung nach Anspruch 2 ist die wenigstens eine Messgröße die Herzfrequenz des Patienten.

Hier erweist es sich als vorteilhaft, dass diese Messgröße von dem Dialysegerät einfach nochmals unabhängig erfasst werden kann. Ebenso kann diese Messgröße von der drahtlos an das Dialysegerät angekoppelten Messeinheit vergleichsweise problemlos erfasst werden.

Bei der Ausgestaltung des Verfahrens nach Anspruch 3 wird die Messung der Messeinrichtung derart durchgeführt, dass die Herzfrequenz aus dem arteriellen und/oder venösen Drucksignal im extrakorporalen Blutkreislauf abgeleitet wird.

Das Messsignal des arteriellen und/oder venösen Drucks ist im Dialysegerät ohnehin vorhanden. Vorteilhaft kann dieses vorhandene Messsignal weiterhin genutzt werden, um daraus die Herzfrequenz zu ermitteln. Hierzu sei weiterhin auf die WO 97/10013 verwiesen. Vorzugsweise wird das arterielle Drucksignal verwendet, da in diesem der Herzpuls stärker ausgeprägt ist.

Vorteilhaft kann die Herzfrequenz über ein bestimmtes Zeitintervall aus dem Drucksignal gemittelt und mit dem gemittelten Wert der Herzfrequenzen verglichen werden, die von der Messeinheit in demselben bestimmten Zeitintervall gemessen und an das Dialysegerät drahtlos übertragen werden. Stimmen diese gemittelten Werte überein, kann geschlossen werden, dass die Messeinheit richtig zugeordnet ist und ordnungsgemäß funktioniert.

Bei der Ausgestaltung des Verfahrens nach Anspruch 4 wird die Messung der Messeinrichtung derart durchgeführt, dass die Herzfrequenz durch eine Manschettenmessung ermittelt wird.

Das Ergebnis der dialysegeräteseitigen Messeinrichtung ist in dem Dialysegerät beispielsweise durch einen konventionellen Blutdruckmonitor verfügbar.

Wenn zum Zeitpunkt t eine Manschettenmessung durchgeführt wird, wird der sich aus der Messung der dialysegeräteseitigen Messeinrichtung ergebende Wert der Herzfrequenz mit dem gemessenen Wert der patientenseitigen drahtlosen Messeinheit verglichen. Der Wert der Messeinheit wird zu demselben Zeitpunkt t ermittelt wie für die Zeitdauer der Manschettenmessung. Der Wert der Messeinheit wird durch eine Mittelwertbildung über dieses Zeitintervall abgeleitet.

Stimmen die Werte der Messeinheit und der Messeinrichtung überein, kann geschlossen werden, dass die Messeinheit richtig zugeordnet ist und ordnungsgemäß funktioniert.

Bei der Ausgestaltung des Verfahrens nach Anspruch 5 ist die wenigstens eine Messgröße der Zeitpunkt bzw. die Zeitpunkte der einzelnen Herzschläge.

Bei der Ausgestaltung nach Anspruch 5 lassen sich die Zeitpunkte der Herzschläge, die von der Messeinrichtung ermittelt werden, z. B. aus dem extrakorporalen Drucksignal ableiten. Weiterhin werden von der Messeinheit die Zeitpunkte ermittelt, zu denen die Herzschläge festgestellt werden. Es erfolgt ein Vergleich, ob die festgestellten Zeitpunkte der Messeinrichtung innerhalb eines Zeitfensters mit den Zeitpunkten der Messeinheit überein stimmen, d.h. ob auf einen Herzschlag genau ein Herzschlag der Messeinheit in konstanter Art und Weise folgt oder voraus geht. Ist dies der Fall, kann geschlossen werden, dass die Messeinheit richtig zugeordnet ist und ordnungsgemäß funktioniert.

Die Auswertung der Zeitpunkte der Herzschläge hat gegenüber der reinen Auswertung der Herzfrequenz den Vorteil, dass Fehlmessungen vermieden werden können, die dadurch entstehen könnten, dass zwei Patienten mit (innerhalb der messtechnischen Toleranzschwellen) identischen Herzfrequenzen behandelt werden, bei denen die Messeinheiten vertauscht wurden. Die Wahrscheinlichkeit, dass bei zwei Patienten nicht nur die Herzfrequenz sondern zusätzlich auch noch die Zeitpunkte der Herzschläge überein stimmen, ist deutlich geringer.

Entsprechend den Verfahrensansprüchen 1 bis 5 sind auch Vorrichtungsansprüche 6 bis 10 formuliert. Die Vorteilsangaben und Erläuterungen zu den Ansprüchen 1 bis 5 gelten entsprechend auch für die Vorrichtungsansprüche 6 bis 10.

Generell kann die Überprüfung am Anfang einer Dialyse vorgenommen werden. Ebenso ist es möglich, diese Überprüfung periodisch - beispielsweise im Abstand von 30 Minuten - zu wiederholen. Diese Überprüfung kann aber auch vergleichsweise problemlos dauernd während der Dialyse vorgenommen werden, weil die entsprechenden Messgrößen problemlos zur Verfügung stehen.

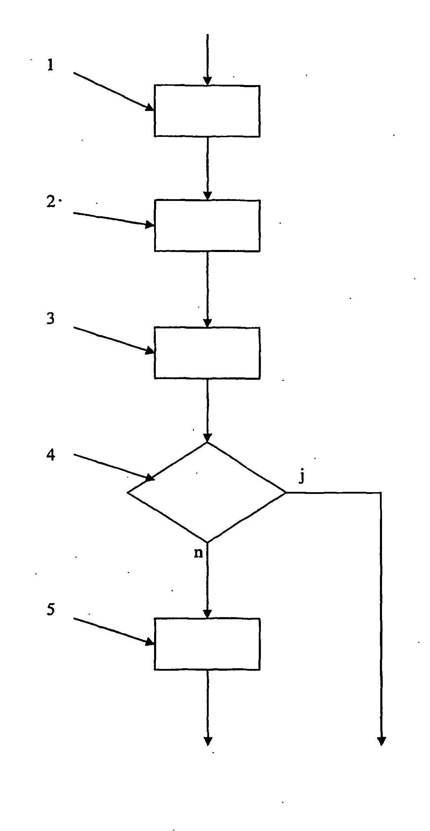

Eine beispielhafte Prinzipdarstellung der Funktionsweise eines erfindungsgemäßen Dialysegerätes ist in der Zeichnung dargestellt.

Entsprechend dem Block 1 wird eine Dialyse gestartet. Die Datenwerte der Messeinrichtung sowie der Messeinheit werden initialisiert, d.h. auf den Wert "0" gesetzt.

Anschließend wird in dem Schritt 2 von der Messeinrichtung, die mit dem Dialysegerät fest verbunden ist, die Messgröße ermittelt.

In dem Schritt 3 wird die entsprechende Messgröße von der Messeinheit ermittelt, die die Daten drahtlos an das Dialysegerät übermittelt.

In dem Schritt 4 wird überprüft, ob der Betrag der Differenz der von der Messeinrichtung ermittelten Messgröße sowie der von der Messeinheit ermittelten entsprechenden Messgröße kleiner ist als ein vorgegebener Schwellwert.

Ist dies der Fall, wird die richtige Zuordnung der Messeinheit zu dem Dialysegerät erkannt.

Andernfalls wird erkannt, dass die Messeinheit eventuell am falschen Patienten angeschlossen sein könnte. Es erfolgt dann entsprechend dem Schritt 5 eine Warnausgabe.

Der Schritt 5 kann noch dahin gehend variiert werden, dass eine Kennung vorgesehen wird, mit der die Fälle gezählt werden, bei denen im Schritt 4 eine Abweichung oberhalb des Schwellwertes festgestellt wurde. Erst wenn diese Kennung eine charakteristische Schwelle erreicht, wird die Fehlermeldung ausgegeben. Damit kann vermieden werden, dass einzelne Fehlmessungen zu Fehlermeldungen führen, die nicht zutreffend sind. Wenn die Werte der Messeinrichtung sowie der Messeinheit dagegen nachfolgend überein stimmen, kann die Kennung sofort oder nach Erfüllung bestimmter Kriterien (z. B. eine Mindestanzahl von übereinstimmenden Messungen) wieder zurück gesetzt werden.

## Patentansprüche

1. Verfahren zur Überprüfung einer einem Dialysegerät zugeordneten Messeinheit, von der wenigstens eine Messgröße erfasst (3) und an das Dialysegerät übertragen wird, wobei die Datenübertragung von der Messeinheit zu dem Dialysegerät drahtlos erfolgt, **dadurch gekennzeichnet, dass** die wenigstens eine Messgröße von einer weiteren, mit dem Dialysegerät fest verbundenen Messeinrichtung ebenfalls erfasst wird (2), wobei aus einem Vergleich der mit der Messeinheit sowie der Messeinrichtung erfassten wenigstens einen Messgröße auf die Zuordnung und/oder Funktion der Messeinheit geschlossen wird (4).

2. Verfahren nach Anspruch 1,
**dadurch gekennzeichnet, dass** die wenigstens eine Messgröße die Herzfrequenz des Patienten ist.

3. Verfahren nach Anspruch 2,
**dadurch gekennzeichnet, dass** die Messung der Messeinrichtung derart durchgeführt wird, dass die Herzfrequenz aus dem arteriellen und/oder venösen Drucksignal im extrakorporalen Kreislauf des Dialysegerätes abgeleitet wird.

4. Verfahren nach Anspruch 2 oder 3,
**dadurch gekennzeichnet, dass** die Messung der Messeinrichtung derart durchgeführt wird, dass die Herzfrequenz durch eine Manschettenmessung ermittelt wird.

5. Verfahren nach einem der Ansprüche 1 bis 4,
**dadurch gekennzeichnet, dass** die wenigstens eine Messgröße der Zeitpunkt bzw. die Zeitpunkte der einzelnen Herzschläge sind.

6. Dialysegerät mit einer dem Dialysegerät zugeordneten Messeinheit, wobei eine Datenübertragung von der Messeinheit zu dem Dialysegerät drahtlos erfolgt, wobei von der Messeinheit wenigstens eine Messgröße erfasst und an das Dialysegerät übertragen wird (3), **dadurch gekennzeichnet, dass** die Messgröße von einer weiteren, mit dem Dialysegerät fest verbundenen Messeinrichtung ebenfalls erfasst wird (2), wobei das Dialysegerät so konfiguriert ist, dass aus einem Vergleich der mit der Messeinheit sowie der Messeinrichtung erfassten wenigstens einen Messgröße auf die Zuordnung und/oder Funktion der Messeinheit geschlossen wird (4).

7. Dialysegerät nach Anspruch 6,
**dadurch gekennzeichnet, dass** die wenigstens eine Messgröße die Herzfrequenz des Patienten ist.

8. Dialysegerät nach Anspruch 7,
**dadurch gekennzeichnet, dass** die Messung der Messeinrichtung derart durchgeführt wird, dass die Herzfrequenz aus dem arteriellen und/oder venösen Drucksignal im extrakorporalen Kreislauf des Dialysegerätes abgeleitet wird.

9. Dialysegerät nach Anspruch 7 oder 8,
**dadurch gekennzeichnet, dass** die Messung der Messeinrichtung derart durchgeführt wird, dass die Herzfrequenz durch eine Manschettenmessung ermittelt wird.

10. Dialysegerät nach einem der Ansprüche 6 bis 9,
**dadurch gekennzeichnet, dass** die wenigstens eine Messgröße der Zeitpunkt bzw. die Zeitpunkte der einzelnen Herzschläge sind.

## Claims

1. Method for checking a measuring unit assigned to a dialyser, by which measuring unit at least one measurable variable is determined (3) and transmitted to the dialyser via a wireless link, **characterised in that** the at least one measurable variable is also determined (2) by another measuring device that is connected physically to the dialyser, a comparison of the values obtained for the at least one measurable variable by the measuring unit and the measuring device indicating whether the measuring unit is the one assigned to the dialyser in question and whether the measuring unit is working properly (4).

2. Method according to claim 1,
**characterised in that** the at least one measurable variable is the patient's pulse rate.

3. Method according to claim 2,
**characterised in that** measurement by the measuring device consists in deriving the pulse rate from the arterial and/or venous pressure signal in the extracorporeal blood circuit of the dialyser.

4. Method according to claim 2 or 3,
**characterised in that** measurement by the measuring device consists in determining the pulse rate by way of a cuff measurement.

5. Method according to one of the claims 1 to 4,
**characterised in that** the at least one measurable variable is the pulse timing.

6. A dialyser with a measuring unit assigned thereto that transmits data to said dialyser via a wireless link, the measuring unit determining and transmitting (3) to the dialyser at least one measurable variable, **characterised in that** the measurable variable is also determined (2) by another measuring device that is connected physically to the dialyser, the dialyser being configured such that a comparison of the values obtained for the at least one measurable variable by the measuring unit and by the measuring device indicates whether the measuring unit is the one assigned to the dialyser in question and/or whether the measuring unit is working properly (4).

7. Dialyser according to claim 6,
**characterised in that** the at least one measurable variable is the patient's pulse rate.

8. Dialyser according to claim 7,
**characterised in that** measurement by the measuring device consists in deriving the pulse rate from the arterial and/or venous pressure signal in the extracorporeal blood circuit of the dialyser.

9. Dialyser according to claim 7 or 8,
**characterised in that** measurement by the measuring device consists in determining the pulse rate by way of a cuff measurement.

10. Dialyser according to one of the claims 6 to 9,
**characterised in that** the at least one measurable variable is the pulse timing.

## Revendications

1. Procédé pour le contrôle d'une unité de mesure associée à un appareil de dialyse, au moins une valeur de mesure (3) étant déterminée par ladite unité de mesure et transmise à l'appareil de dialyse, la transmission des données de l'unité de mesure à l'appareil de dialyse étant réalisée sans fil, **caractérisé en ce que** la au moins une valeur de mesure est déterminée également par un dispositif de mesure supplémentaire relié fixement à l'appareil de dialyse (2), l'attribution et/ou la fonction de l'unité de mesure étant déterminée(s) à partir d'une comparaison de la au moins une valeur de mesure déterminée par l'unité de mesure et par le dispositif de mesure (4).

2. Procédé selon la revendication 1, **caractérisé en ce que** la au moins une valeur de mesure est la fréquence cardiaque du patient.

3. Procédé selon la revendication 2, **caractérisé en ce que** la mesure exécutée par le dispositif de mesure est réalisée de telle sorte que la fréquence cardiaque est déduite du signal de pression artériel ou veineux dans la circulation sanguine extracorporelle de l'appareil de dialyse.

4. Procédé selon la revendication 2 ou 3, **caractérisé en ce que** la mesure exécutée par le dispositif de mesure est réalisée de telle sorte que la fréquence cardiaque est mesurée à l'aide d'une mesure par manchon gonflable.

5. Procédé selon l'une des revendications 1 à 4, **caractérisé en ce que** la au moins une valeur de mesure est le ou les moments de chaque battement de coeur.

6. Appareil de dialyse avec une unité de mesure associée à l'appareil de dialyse, la transmission des données de l'unité de mesure vers l'appareil de dialyse étant réalisé sans fil, au moins une valeur de mesure étant déterminée par l'unité de mesure et transmise à l'appareil de dialyse (3), **caractérisé en ce que** la valeur de mesure est également déterminée par un appareil de mesure supplémentaire relié fixement à l'appareil de dialyse (2), l'appareil de dialyse étant configuré de telle sorte que l'attribution et/ou la fonction de l'unité de mesure est/sont déterminée(s) à partir d'une comparaison de la au moins une valeur de mesure déterminée par l'unité de mesure et par le dispositif de mesure (4).

7. Appareil de dialyse selon la revendication 6, **caractérisé en ce que** la au moins une valeur de mesure est la fréquence cardiaque du patient.

8. Appareil de dialyse selon la revendication 7, **caractérisé en ce que** la mesure exécutée par le dispositif de mesure est réalisée de telle sorte que la fréquence cardiaque est déduite du signal de pression artériel ou veineux dans la circulation sanguine extracorporelle de l'appareil de dialyse.

9. Appareil de dialyse selon l'une des revendications 7 ou 8, **caractérisé en ce que** la mesure exécutée par le dispositif de mesure est réalisée de telle sorte que la fréquence cardiaque est mesurée à l'aide d'une mesure par manchon gonflable.

10. Appareil de dialyse selon l'une des revendications 6 à 9, **caractérisé en ce que** la au moins une valeur de mesure est le ou les moments de chaque battement de coeur.
